Europäisches Patentamt

(19) European Patent Office

Office européen des brevets

(11) EP 0 667 771 B1

(12) **FASCICULE DE BREVET EUROPEEN**

(45) Date de publication et mention
de la délivrance du brevet:
**15.04.1998 Bulletin 1998/16**

(21) Numéro de dépôt: **93924682.3**

(22) Date de dépôt: **08.11.1993**

(51) Int. Cl.$^6$: **A61K 31/335**

(86) Numéro de dépôt international:
**PCT/FR93/01096**

(87) Numéro de publication internationale:
**WO 94/10995 (26.05.1994 Gazette 1994/12)**

(54) **COMPOSITIONS ANTITUMORALES CONTENANT DES DERIVES DU TAXANE**

ANTITUMORZUSAMMENSETZUNGEN ENTHALTEND TAXANDERIVATE

ANTITUMOUR COMPOSITIONS CONTAINING TAXANE DERIVATIVES

(84) Etats contractants désignés:
**AT BE CH DE DK ES FR GB GR IE IT LI LU NL PT SE**

(30) Priorité: **10.11.1992 FR 9213525**

(43) Date de publication de la demande:
**23.08.1995 Bulletin 1995/34**

(60) Demande divisionnaire:
**97117252.3 / 0 827 745**

(73) Titulaire:
**RHONE-POULENC RORER S.A.**
**92160 Antony (FR)**

(72) Inventeur:
**BISSERY, Marie-Christine**
**F-94400 Vitry-sur-Seine (FR)**

(74) Mandataire: **Pilard, Jacques**
**RHONE-POULENC RORER S.A.**
**Direction Brevets**
**20 avenue Raymond Aron**
**92165 Antony Cédex (FR)**

(56) Documents cités:
• **CHEMICAL PATENTS INDEX, DOCUMENTATION ABSTRACTS JOURNAL Section Ch, Week 9210, Derwent Publications Ltd., London, GB; Class B05, AN 92-079710 & US,A,7 696 923 (US DEPT. HEALTH) 21 Janvier 1992 & WO,A,92 19765 (US DEPT. HEALTH) 12 Novembre 1992**

Remarques:
• Demande divisionnaire 97117252.3 déposée le 06/10/97.
• Le dossier contient des informations techniques présentées postérieurement au dépôt de la demande et ne figurant pas dans le présent fascicule.

**Description**

La présente invention concerne les combinaisons du taxol, du Taxotère et de leurs analogues et de substances thérapeutiquement utiles dans le traitement des maladies néoplastiques.

Le taxol, le Taxotère et leurs analogues, qui présentent des propriétés antitumorales et antileucémiques remarquables, sont particulièrement utiles dans le traitement des cancers de l'ovaire, du sein ou du poumon.

La préparation du taxol, du Taxotère et de leurs dérivés font l'objet, par exemple, des brevets européens EP 0 253 738 et EP 0 253 739 et de la demande internationale PCT WO 9209589.

Généralement les doses utilisées, qui dépendent des facteurs propres au sujet à traiter, sont comprises entre 1 et 10 mg/kg par voie intra-péritonéale ou entre 1 et 3 mg/kg par voie intra-veineuse.

Le brevet US-A-7 696 923 décrit une méthode de tests pour déterminer les méthodes de traitement anticancéreuses lors de l'association de plusieurs molécules anticancéreuses agissant sur différentes phases du cycle de développement des cellules de mammifères. Cette invention décrit des techniques de diagnostic pour mesurer l'activité de substances anticancéreuses.

Le brevet publié sous le n° WO 92/19765 correspond au texte précédent complété par des exemples de test sur des cellules génétiquement transformées de chacune des substances anticancéreuses utilisées seule. La seule association de substances anticancéreuses décrite est celle concernant le cis-platine associé au taxol (exemple 5).

Il a maintenant été trouvé, et c'est ce qui fait l'objet de la présente invention, que l'efficacité du taxol, du Taxotère et de leurs analogues peut être considérablement améliorée lorsqu'ils sont administrés en association avec au moins une substance thérapeutiquement utile dans les traitements anticancéreux ayant un mécanisme d'action identique à celui des dérivés du taxane.

Parmi les substances qui peuvent être utilisées en association ou en combinaison avec le taxol, le Taxotère ou leurs analogues peuvent être cités des des poisons du fuseau dont les alcaloïdes de vinca tels que la vinblastine ou la vincristine ou leurs analogues de synthèse tels que la navelbine, ou l'estramustine ou les taxoïdes, des épidophyllotoxines tels que l'étoposide ou le teniposide, des antibiotiques tels que la daunorubicine, la doxorubicine, la bléomycine ou la mitomycine, des inhibiteurs de topoisomérase tels que les dérivés de la camptothécine choisis parmi le CPT-11 et le topotécan ou des dérivés du pyridobenzoindole.

Par ailleurs, l'activité des produits dépendant des doses utilisées, il est possible d'utiliser des doses plus élevées et d'augmenter l'activité en diminuant les phénomènes de toxicité ou en retardant leur apparition en associant au taxol, au Taxotère, à leurs analogues ou à leurs combinaisons avec d'autres substances thérapeutiquement actives des facteurs de croissance de type hématopoïétique tels que le G-CSF ou le GM-CSF ou certaines interleukines.

Les combinaisons ou les associations selon l'invention permettent d'éviter ou de retarder les phénomènes de résistance pléïotropique ou de "multi-drug resistance".

Plus particulièrement, l'invention concerne les associations du taxol, du Taxotère et de leurs analogues avec les alcaloïdes de vinca, la doxorubicine et l'étoposide.

L'efficacité améliorée d'une combinaison selon l'invention peut être mise en évidence par la détermination du synergisme thérapeutique.

L'efficacité d'une combinaison selon l'invention peut aussi être caractérisée par l'addition des actions de chaque constituant.

Une combinaison manifeste un synergisme thérapeutique s'il est thérapeutiquement supérieur à l'un ou l'autre des constituants utilisé à sa dose optimale [T.H. CORBETT et coll., Cancer Treatment Reports, 66, 1187 (1982)].

Pour mettre en évidence l'efficacité d'une combinaison, il peut être nécessaire de comparer la dose maximale tolérée de la combinaison à la dose maximale tolérée de chacun des constituants isolés dans l'étude considérée. Cette efficacité peut être quantifiée, par exemple par le $\log_{10}$ des cellules tuées qui est déterminé selon la formule suivante :

$$\log_{10} \text{ des cellules tuées} = \text{T-C (jours)}/3.32 \cdot T_d$$

dans laquelle T - C représente le délai de croissance des cellules qui est le temps moyen, en jours, pour que les tumeurs du groupe traité (T) et les tumeurs du groupe témoin (C) aient atteint une valeur prédéterminée (1 g par exemple) et $T_d$ représente le temps, en jours, nécessaire au doublement du volume de la tumeur chez les animaux témoins.[T.H. CORBETT et coll., Cancer, 40, 2660.2680 (1977) ; F.M. SCHABEL et coll., Cancer Drug Development, Part B, Methods in Cancer Research, 17, 3-51, New-York, Academic Press Inc. (1979)] Un produit est considéré comme actif si $\log_{10}$ des cellules tuées est supérieur ou égal à 0,7. Un produit est considéré comme très actif si $\log_{10}$ des cellules tuées est supérieur à 2,8.

La combinaison, utilisée à sa dose maximale tolérée propre, dans laquelle chacun des constituants sera présent à une dose généralement inférieure ou égale à sa dose maximale tolérée, manifestera une synergie thérapeutique lorsque le $\log_{10}$ des cellules tuées sera supérieur à la valeur du $\log_{10}$ des cellules tuées du meilleur constituant lorsqu'il est administré seul.

L'efficacité des combinaisons sur les tumeurs solides peut être déterminée expérimentalement de la manière suivante :

Les animaux soumis à l'expérience, généralement des souris, sont greffés bilatéralement par voie sous-cutanée avec 30 à 60 mg d'un fragment de tumeur au jour 0. Les animaux portant les tumeurs sont mélangés avant d'être soumis aux divers traitements et contrôles. Dans le cas de traitement de tumeurs avancées, on laisse les tumeurs se développer jusqu'à la taille désirée, les animaux ayant des tumeurs insuffisamment développées étant éliminés. Les animaux sélectionnés sont répartis au hasard pour subir les traitements et les contrôles. Des animaux non porteurs de tumeurs peuvent être également soumis aux mêmes traitements que les animaux porteurs afin de pouvoir dissocier l'effet toxique de l'effet propre sur la tumeur. La chimiothérapie commence généralement de 3 à 22 jours après le greffage selon le type de tumeur et les animaux sont observés tous les jours. Les différents groupes d'animaux sont pesés trois ou quatre fois par semaine jusqu'à ce que la perte maximale de poids soit atteinte puis les groupes sont pesés au moins une fois par semaine jusqu'à la fin de l'essai.

Les tumeurs sont mesurées deux ou trois fois par semaine jusqu'à ce que la tumeur atteigne environ 2 g ou jusqu'à la mort de l'animal si celle-ci survient avant que la tumeur atteigne 2 g. Les animaux sont autopsiés lors du sacrifice.

L'activité antitumorale est déterminée en fonction des différents paramètres enregistrés.

Pour l'étude des combinaisons sur des leucémies, les animaux sont greffés avec un nombre déterminé de cellules et l'activité antitumorale est déterminée par l'augmentation du temps de survie des souris traitées par rapport aux témoins. Un produit est considéré comme actif si le temps d'augmentation de survie est supérieur à 27 % et il est considéré comme très actif s'il est supérieur à 75 % dans le cas de la leucémie P388.

A titre d'exemples, sont donnés, dans les tableaux suivants les résultats obtenus avec des combinaisons du Taxotère et de divers agents chimiothérapeutiques tels que l'étoposide (agent d'hémisynthèse de la podophyllotoxine) et la vincristine (alcaloïde de vinca) utilisées à leur dose optimale.

TABLEAU 1

| Activité de la combinaison Taxotère + étoposide à la dose optimale sur le mélanome B16 précoce greffé par voie sous-cutanée | | | | |
|---|---|---|---|---|
| Produit | Dose mg/kg/injection i. v. | Administration aux jours : | Dose totale mg/kg | $\log_{10}$ cell. tuées |
| Taxotère | 17,5 | 4, 7, 10, 13 | 70 | 2,8 |
| étoposide | 46,2 | 4, 7, 10, 13 | 184,8 | 2,8 |
| Taxotère + étoposide | 15,7 | 4, 7, 10, 13 (simultanée) | 62,8 | 4,1 |
| | 13,8 | | 55,2 | |

TABLEAU 2

| Activité de la combinaison Taxotère + vincristine à la dose optimale sur la leucémie P388 ($10^6$ cellules i.p.) | | | | |
|---|---|---|---|---|
| Produit | Dose mg/kg/injection i. v. | Administration aux jours : | Dose totale mg/kg | Augmentation du temps de survie (%) |
| Taxotère | 21,7 | 1, 4, 7 | 65,1 | 15 |
| Vincristine | 1,2 | 1, 4, 7 | 3,6 | 46 |
| Taxotère + vincristine | 21,75 | 1, 4, 7 (simultanée) | 65,25 | 62 |
| | 1,2 | | 3,6 | |
| Taxotère + Vincristine | 21,75 | 1, 4, 7 (à 4 heures d'intervalle) | 65,25 | 77 |
| | 1,2 | | 3,6 | |

La présente invention concerne également les compositions pharmaceutiques contenant les combinaisons selon l'invention.

Les produits qui constituent la combinaison peuvent être administrés simultanément, séparément ou d'une manière étalée dans le temps de façon à obtenir le maximum d'efficacité de la combinaison ; chaque administration pouvant avoir une durée variable allant d'une administration totale rapide à une perfusion continue.

Il en résulte que, au sens de la présente invention, les combinaisons ne sont pas uniquement limitées à celles qui sont obtenues par association physique des constituants, mais aussi à celles qui permettent une administration séparée qui peut être simultanée ou étalée dans le temps.

Les compositions selon l'invention sont de préférence les compositions administrables par voie parentérale. Cependant, ces compositions peuvent être administrées par voie orale ou par voie intrapéritonéale dans le cas des thérapies locorégionales.

Les compositions pour administration parentérale sont généralement des solutions ou des suspensions stériles pharmaceutiquement acceptables qui peuvent éventuellement être préparées extemporanément au moment de l'emploi. Pour la préparation de solutions ou de suspensions non aqueuses peuvent être utilisées des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution du produit dans l'eau. Les solutions aqueuses conviennent pour l'administration intraveineuse dans la mesure où le pH est convenablement ajusté et où l'isotonicité est réalisée, par exemple par une quantité suffisante de chlorure de sodium ou de glucose. La stérilisation peut être réalisée par chauffage ou par tout autre moyen qui n'altère pas la composition. Les combinaisons peuvent aussi se présenter sous forme de liposomes ou sous forme d'association avec des supports tels que les cyclodextrines ou les polyéthylèneglycols.

Les compositions pour administration orale ou intrapéritonéale sont, de préférence, des solutions ou des suspensions aqueuses.

Dans les combinaisons selon l'invention dont l'application des constituants peut être simultanée, séparée ou étalée dans le temps, il est particulièrement avantageux que la quantité de dérivé du taxane représente de 10 à 90 % en poids de la combinaison cette teneur pouvant varier en fonction de la nature de la substance associée, de l'efficacité recherchée et de la nature du cancer à traiter.

Les combinaisons selon l'invention sont particulièrement utiles dans le traitement des cancers du sein, de l'ovaire ou du poumon. En particulier, elles peuvent présenter l'avantage de pouvoir mettre en oeuvre les constituants à des doses nettement plus faibles que celles auxquelles ils sont utlilisés seuls.

L'exemple suivant illustre une combinaison selon l'invention.

EXEMPLE

On prépare selon la technique habituelle, pour l'administration intraveineuse, des ampoules de 10 cm3 contenant 100 mg de Taxotère.

On prépare selon la technique habituelle, pour l'administration intraveineuse des ampoules de 5 cm3 contenant 100 mg d'étoposide.

Ces solutions sont administrées simultanément, après dilution convenable, par perfusion.

Le traitement peut être répété plusieurs fois par jour ou par semaine jusqu'à une rémission partielle ou totale ou une guérison.

**Revendications**

1.  Combinaisons du taxol, du Taxotère et de leurs analogues avec des poisons du fuseau, des épipodophyllotoxines, des antibiotiques, des inhibiteurs de topoisomérase.

2.  Combinaisons selon la revendication 1 caractérisées en ce que les poisons du fuseau sont choisis parmi les alcaloïdes de vinca, leurs analogues synthétiques ou semi-synthétiques, ou l'estramustine ou les taxoïdes.

3.  Combinaisons selon la revendication 1 caractérisées en ce que les épipodophyllotoxines sont choisies parmi l'étoposide et le teniposide.

4.  Combinaisons selon la revendication 1 caractérisées en ce que les antibiotiques sont choisis parmi la daunorubicine, la doxorubicine, la bléomycine ou la mitomycine.

5.  Combinaisons selon la revendication 1 caractérisées en ce que les inhibiteurs de topoisomérase sont choisis parmi la camptothécine et ses dérivés choisis parmi le CPT-11 et le topotécan et les dérivés du pyridobenzoindole.

6.  Combinaisons selon l'une des revendications 1 à 5 caractérisées en ce qu'elles contiennent en outre des facteurs de croissance de type hématopoïétiques.

7. Combinaisons selon l'une des revendications 1 à 6 caractérisées en ce qu'elles contiennent de 10 à 90 % en poids de taxol, de Taxotère ou de leurs analogues.

8. Produits contenant du taxol, du Taxotère ou leurs analogues et au moins une substance thérapeutiquement utile telle que définie dans l'une des revendications 1 à 7 dans le traitement des maladies néoplastiques comme préparation combinée pour une utilisation simultanée, séparée ou étalée dans le temps en thérapie anticancéreuse.

**Claims**

1. Combinations of taxol, Taxotere and their analogues with spindle poisons, epipodophyllotoxins, antibiotics and topoisomerase inhibitors.

2. Combinations according to claim 1, characterized in that the spindle poisons are chosen from vinca alkaloids, their synthetic or semi-synthetic analogues, or estramustine or taxoids.

3. Combinations according to claim 1, characterized in that the epipodophyllotoxins are chosen from etoposide and teniposide.

4. Combinations according to claim 1, characterized in that the antibiotics are chosen from daunorubicin, doxorubicin, bleomycin and mitomycin.

5. Combinations according to claim 1, characterized in that the topoisomerase inhibitors are chosen from camptothecin and its derivatives chosen from CPT-11 and topotecan and pyridobenzoindole derivatives.

6. Combinations according to one of claims 1 to 5, characterized in that they contain, in addition, growth factors of the haematopoietic type.

7. Combinations according to one of claims 1 to 6, characterized in that they contain from 10 to 90 % by weight of taxol, Taxotere or their analogues.

8. Products containing taxol, Taxotere or their analogues and at least one substance which is therapeutically useful, as defined in one of claims 1 to 7, in the treatment of neoplastic diseases, as a combined preparation for use simultaneously, separately or spaced out over a period of time in anticancer therapy.

**Patentansprüche**

1. Kombinationen aus Taxol, Taxotere und ihren Analoga mit Mitosegiften, Epipodophyllotoxinen, Antibiotika, Topoisomeraseinhibitoren.

2. Kombinationen nach Anspruch 1, dadurch **gekennzeichnet**, daß die Mitosegifte aus Vinca-Alkaloiden, ihren synthetischen oder semisynthetischen Analoga oder Estramustin oder Taxoiden ausgewählt sind.

3. Kombinationen nach Anspruch 1, dadurch **gekennzeichnet**, daß die Epipodophyllotoxine aus Etoposid oder Teniposid ausgewählt sind.

4. Kombinationen nach Anspruch 1, dadurch **gekennzeichnet**, daß die Antibiotika aus Daunorubicin, Doxorubicin, Bleomycin oder Mitomycin ausgewählt sind.

5. Kombinationen nach Anspruch 1, dadurch **gekennzeichnet**, daß die Topoisomeraseinhibitoren aus Camptothecin und seinen Derivaten, ausgewählt aus CPT-11 und Topotecan, und den Pyridobenzoindolderivaten ausgewählt sind.

6. Kombinationen nach einem der Ansprüche 1 bis 5, dadurch **gekennzeichnet**, daß sie weiterhin Wachstumsfaktoren vom hämatopoietischen Typ enthalten.

7. Kombinationen nach einem der Ansprüche 1 bis 6, dadurch **gekennzeichnet**, daß sie 10 bis 90 Gew.-% Taxol, Taxotere oder ihre Analoga enthalten.

8. Produkte, die Taxol, Taxotere oder ihre Analoga und mindestens eine therapeutisch zur Behandlung von neoplastischen Krankheiten nützliche Substanz nach einem der Ansprüche 1 bis 7 enthalten, als Kombinationspräparat zur zeitlich gleichzeitigen, getrennten oder abgestuften Verwendung in der Antikrebstherapie.